(19) Europäisches Patentamt
European Patent Office
Office européen des brevets

(11) Veröffentlichungsnummer: **0 088 050**
**B1**

(12) # EUROPÄISCHE PATENTSCHRIFT

(45) Veröffentlichungstag der Patentschrift: **03.09.86**

(51) Int. Cl.⁴: **G 03 C 1/68**, C 09 D 11/10

(21) Anmeldenummer: **83810075.8**

(22) Anmeldetag: **21.02.83**

(54) **Photohärtbare gefärbte Massen.**

(30) Priorität: **26.02.82 CH 1196/82**

(43) Veröffentlichungstag der Anmeldung: **07.09.83 Patentblatt 83/36**

(45) Bekanntmachung des Hinweises auf die Patenterteilung: **03.09.86 Patentblatt 86/36**

(84) Benannte Vertragsstaaten: **BE CH DE FR GB IT LI NL**

(56) Entgegenhaltungen:
**EP-A-0 003 002**
**EP-A-0 007 468**
**EP-A-0 036 075**
**US-A-2 759 970**
**US-A-3 910 916**
**US-A-4 124 722**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Berner, Godwin, Dr., Waldhofstrasse 70, CH- 4310 Rheinfelden (CH)**
Erfinder: **Hüsler, Rinaldo, Dr., Belchenstrasse 12, CH- 4054 Basel (CH)**
Erfinder: **Kirchmayr, Rudolf, Dr., Ettingerstrasse 9, CH- 4147 Aesch (CH)**

EP 0 088 050 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft photohärtbare gefärbte Massen, die ein olefinisch ungesättigtes Bindemittel, ein Pigment oder einen Farbstoff und einen spezifischen Photoinitiator enthalten. Der Photoinitiator ist ein aromatisch-aliphatisches Keton, das im aromatischen Teil eine Thioethergruppe enthält und dessen aliphatischer Teil ein tertiäres α-C-Atom enthält, an welchem sich eine Aninogruppe befindet.

Es ist bekannt, dass man zur Beschleunigung der Photohärtung von gefärbten Massen, wie beispielsweise Druckfarben oder Anstrichstoffen, vor der Bestrahlung Photoinitiatoren zusetzt. Auf diese Weise ist es möglich, solche Massen in sehr kurzer Bestrahlungszeit so weit zu härten, dass ihre Oberfläche nicht mehr klebrig ist. Während es für transparente Ueberzugsmassen eine Reihe von technisch befriedigenden Photoinitiatoren gibt, stellt die Strahlenhärtung gefärbter Massen wegen der Anwesenheit der Licht-absorbierenden Pigmente oder Farbstoffe ein besonders schwierig zu lösendes Problem dar. Bei Druckfarben kommt dazu die Forderung nach extrem kurzen Härtungszeiten wegen der hohen Geschwindigkeit moderner Druckmaschinen. Die Anforderungen an Photoinitiatoren für gefärbte Massen sind deshalb wesentlich höher als für transparente photohärtbare Massen.

Bisher in der Technik verwendete Photoinitiatoren für die Härtung solcher gefärbter Massen wie z.B. Druckfarben oder Anstrichstoffe sind meist synergistische Gemische von ketonischen Photoinitiatoren mit spezifischen Aminen, beispielsweise Gemisch von Benzophenon mit Michlers Keton (4,4'-Bis-dimethylaminobenzophenon) oder mit p-Dimethylaminobenzoesäurealkylestern oder Gemische von Thioxanthonen mit N-Methyl-diethanolamin. Solche Keton-Amin-Gemische neigen zur Vergilbung im Licht. Dies kann sich bereits bei der Strahlenhärtung äussern, spatestens aber bei längerer Lichteinwirkung auf die gehärteten Schichten. Einige dieser Verbindungen sind in den üblichen Acrylharz-Bindemitteln schwer löslich, neigen zur Rekristallisation und verkürzen die Lagerfähigkeit der Gemische beträchtlich. Andere solcher Verbindungen, wie z.B. die Alkanolamine, sind wasserlöslich und daher für Nass-Offset-Druckfarben nicht verwendbar. Ferner wirken die Keton-Amin-Gemische nach einem bimolekularen Startmechanismus, der diffusionskontrolliert ist und daher in höherviskosen Systemen relativ langsam abläuft.

In der EP-Anmeldung, Publ. Nr. 3002, wurden bereits molekulare Kombinationen von Arylketonen und Aminen als Photoinitiatoren vorgeschlagen, deren Aminogruppe sich an einem tertiären C-Atom in α-Stellung zur Carbonylgruppe befindet. Die dort beschriebenen Aminoketone erwiesen sich jedoch als Photoinitiatoren in Klarlacken den entsprechenden Hydroxyketonen, die in derselben Patentschrift beschrieben sind, als unterlegen. Die dort beschriebenen Hydroxyketone sind zwar hervorragende Initiatoren für transparente Lacke, zeigen aber in pigmentierten Massen, wie z.B. in Druckfarben, nur mässig gute Wirkung.

Ueberraschenderweise wurde gefunden, dass solche Aminoketone, die am aromatischen Kern eine Thioether-Gruppe tragen, in gefärbten Massen, insbesondere in Druckfarben, eine hervorragende Initiatorwirkung zeigen und die Nachteile der Keton-Amin-Gemische nicht oder in wesentlich geringerem Ausmass besitzen.

Gegenstand der Erfindung sind daher photohärtbare gefärbte Massen, enthaltend

a) ein olefinisch ungesättigtes, photopolymerisierbares Bindemittel,

b) ein Pigment oder einen Farbstoff und

c) einen Photoinitiator, dadurch gekennzeichnet, dass der Photoinitiator eine Verbindung der Formel 1 ist

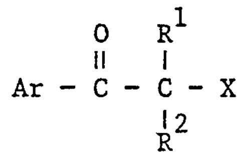

I,

worin Ar ein durch die Gruppe -S-$R^9$ substituierter Phenylrest ist, $R^9$ Wasserstoff, $C_1$-$C_8$-Alkyl, $C_3$-$C_6$-Alkenyl, Cyclohexyl, Benzyl, Phenyl, Tolyl oder eine der Gruppen $-CH_2CH_2OH$, $-CH_2CH_2-OOC-CH=CH_2$, $-CH_2-COO(C_1-C_4\text{-Alkyl})$, $-CH_2CH_2-COO(C_1-C_4\text{-Alkyl})$,

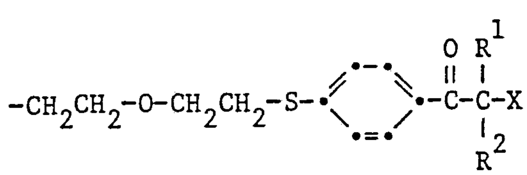

oder

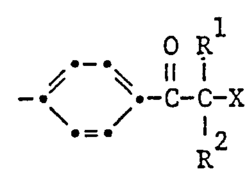

bedeutet,

$R^1$ und $R^2$ $C_1$-$C_4$-Alkyl oder $R^1$ und $R^2$ zusammen $C_4$-$C_5$-Alkylen bedeuten und

X ein Morpholinorest oder ein Rest der Formel $-N(CH_2CH_2OCH_3)_2$ist.

Besonders bevorzugt sind Photoinitiatoren der Formel I, worin Ar 4-Mercaptophenyl, 4-Methylthiophenyl oder 4-(2-Hydroxy-ethyl)thiophenyl ist, $R^1$ und $R^2$ unabhängig voneinander Methyl, Ethyl oder Butyl sind und X eine Morpholinogruppe bedeutet.

Beispiele für einzelne Verbindungen der Formel 1 sind die folgenden Verbindungen:
2-Methyl-1-[4-(methylthio)phenyl]-2-morpholino-propanon-1,
2-Methyl-1-[4-(ethylthio)phenyl]-2-morpholino-propanon-1,
2-Methyl-1-[4-(butylthio)phenyl]-2-morpholino-propanon-1
2-Methyl-1-[4-(octylthio)phenyl]-2-morpholino-propanon-1
2-Methyl-1[4-mercaptophenyl]-2-morpholino-propanon-1
2-Methyl-1-[4-(2-hydroxyethylthio)phenyl]-2-morpholino-propanon-1
2-Methyl-1-[4-(2-acryloyloxyethylthio)phenyl]-2-morpholino-propanon-1
2-Methyl-1-[4-(phenylthio)phenyl]-2-morpholino-propanon-1
2-Methyl-1-[4-(methoxycarbonylmethylthio)phenyl]-2-morpholino-propanon-1
2-Methyl-1-[4-(methylthio)phenyl]-2-di(2-methoxyethyl)amino-propanon-1
2-Methyl-1-[4-(phenylthio)phenyl]-2-di(2-methoxyethyl)amino-propanon-1
4,4'-Bis(α-morpholino-isobutyroyl)diphenylsulfid
2,2'-Bis[4-(α-morpholino-isobutyroyl]-phenylthio)diethylether
2-Ethyl-1-[4-(methylthio)phenyl]-2-morpholino-hexanon-1
1-(4-Methylthiobenzoyl)-1-morpholino-cyclohexan
2-Methyl-1-[4-(methylthio)phenyl]-2-morpholino-butanon-1
2-Methyl-1-[4-(methylthio)phenyl]-2-morpholino-pentanon-1
2-Ethyl-1-[4-(methylthio)phenyl]-2-morpholino-butanon-1
2-Propyl-1-[4-(methylthio)phenyl]-2-morpholino-pentanon-1
2-Methyl-1-[4-(2-ethoxycarbonylethylthio)phenyl]-2-morpholino-propanon-1
2-Methyl-1-[4-(benzylthio)phenyl]-2-morpholino-propanon-1
2-Methyl-1-[4-(cyclohexylthio)phenyl]-2-morpholino-propanon-1
2-Methyl-1-[4-(ethoxycarbonylmethylthio)phenyl]-2-morpholino-propanon-1
2-Methyl-1-[3-(methylthio)phenyl]-2-morpholino-propanon-1
2-Methyl-1-[2-(methylthio)phenyl]-2-morpholino-propanon-1
2-Ethyl-1-[4-(isopropylthio)phenyl]-2-morpholi-no-butanon-1
2-Methyl-1-[4-(allylthio)phenyl]-2-morpholino-propanon-1
2-Methyl-1-[4-(methallylthio)phenyl]-2-morpholino-propanon-1
1-(4-Methylthiobenzoyl)-1-morpholino-cyclopentan
2-Methyl-1-[4-(4-tolylthio)phenyl]-2-morpholino-propanon-1

Die Verbindungen der Formel I sind zum Teil bekannte Verbindungen, deren allgemeine Herstellung in der Europ. Patentanmeldung Publ. Nr. 3002 beschrieben ist. Soweit die Verbindungen neu sind, können sie in Analogie dazu hergestellt werden.

Nähere Einzelheiten zur Synthese von Verbindungen der Formel I können den folgenden Herstellungsbeispielen entnommen werden. Die Temperaturen sind darin in °C angegeben.

**Beispiel A**

Herstellung von Aminoketon-thioethern über die α-Halogenketon-thioether:

$A_1$) 2-Brom-2-methyl-1-[4-(methylthio)phenyl]-propanon-1

369,2 g (1,9 Mol) 2-Methyl-1-[4-(methylthio)phenyl]-propanon 1 werden in 400 ml Tetrachlorkohlenstoff gelöst. Zu dieser Lösung werden bei Raumtemperatur unter Kühlung 303,7 g (1,9 Mol) Brom, verdünnt mit 270 ml Tetrachlorkohlenstoff, langsam zugetropft. Dann wird mit Stickstoff das gelöste HBr-Gas ausgeblasen. Die Lösung wird eingeengt und anschliessend wie nachfolgend beschrieben weiter umgesetzt.

$A_2$) 3,3-Dimethyl-2-methoxy-2-[4-(methylthio)phenyl]-oxiran

95,1 g (1,76 Mol) Natriummethylat werden in 600 ml Methanol gelöst und zu dieser Lösung werden bei Rückflusstemperatur 437,1 g (1,60 Mol) 2-Brom-2-methyl-1-[4-(methylthio)phenyl]-propanon-1, gelöst in 300 ml Methanol, zugetropft. Dann wird das Methanol abdestilliert. Der Rückstand wird auf Eiswasser gegossen und mit Diethylether extrahiert. Die Etherlösung wird mit Wasser gewaschen, über $Na_2SO_4$ getrocknet und eingeengt. Die erhaltenen Kristalle werden durch Vakuumdestillation gereinigt.

Smp. 62-64°C Sdp. 90 - 93°C/14 Pa.

$A_3$) 2-Methyl-1-[4-(methylthio)phenyl]-2-morpholino-propanon-1

151,4 g (0,675 Mol) 3,3-Dimethyl-2-methoxy-2-[4-(methylthio)phenyl]-oxiran (Smp. 62-64°C) werden in 235,2 g (2,70 Mol) Morpholin gelöst und auf Rückfluss erwärmt.

Nach 15 Stunden wird abgekühlt und das Morpholin abdestilliert. Der Rückstand (Smp. 68-71°C) wird in Diethylether aufgenommen und mit verdünnter Salzsäure extrahiert. Die Salzsäurelösung wird alkalisch gestellt und mit Ether extrahiert. Die Etherlösung wird mit $Na_2SO_4$ getrocknet und eingeengt. Der Rückstand kann aus Ethanol umkristallisiert werden.

Smp. 75-76°C.

$C_{15}H_{21}NO_2S$ (279,40)

berechnet: C 64,48 % H 7,58 % N 5,01 % O 11,45 % S 11,48 %

gefunden: C 64,49 % H 7,51 % N 5,10 % O 11,58 % S 11,51 %

In gleicher Weise werden weitere Aminderivate hergestellt, die in der folgenden Tabelle 1 aufgeführt sind. Bei tiefsiedenden Aminen wird unter Druck gearbeitet. Die erhaltenen Rohprodukte können durch Umkristallisation oder durch Chromatographie über einer Kieselgel-Mitteldrucksäule gereinigt werden (Fliessmittel; Gemische von Essigester und Hexan).Von allen Verbindungen wurde die Struktur durch ein H-NMR-Spektrum bestätigt.

**Beispiel B**

Herstellung von Aminoketon-thioethern über die Halogenaryl-aminoketone

$B_1$) 2-Chlor-1-(4-chlorphenyl)-2-methyl-propanon-1

182,7 g (1,0 Mol) 1-(4-Chlorphenyl)-2-methyl-propanon-1 werden auf 40° erwärmt und mit 71 g (1,0 Mol) Chlorgas in 5 Stunden bei 40 - 65° chloriert. Dann wird mit Stickstoff das gelöste HCl-Gas ausgeblasen. Das flüssige Rohprodukt wird anschliessend weiter umgesetzt.

$B_2$) 2-(4-Chlorphenyl)-3,3-dimethyl-2-methoxy-oxiran

57,0 g (1,056 Mol) Natriummethylat werden in 360 ml Methanol gelöst und zu dieser Lösung werden bei Rückflusstemperatur 208,4 g (0,96 Mol) 2-Chlor-1-(4-chlorphenyl)-2-methyl-propanon-1 zugetropft. Dann wird das Methanol abdestilliert. Der Rückstand wird auf Eiswasser gegossen und mit Diethylether extrahiert. Die Etherlösung wird mit Wasser gewaschen, über $Na_2SO_4$ getrocknet und eingeengt. Das erhaltene Oel wird durch Vakuumdestillation gereinigt.

Sdp. 107°C/1,3 KPa

$C_{11}H_{13}ClO_2$ (212,68)

berechnet: C 62,12 % H 6,16 % Cl 16,67 %

gefunden: C 61,89 % H 6,17 % Cl 16,61 %

$B_3$) 1-(4-Chlorphenyl)-2-methyl-2-morpholino-propanon-1

85 1 g (0,4 Mol) 2-(4-Chlorphenyl)-3,3-dimethyl-2-methoxy-oxiran und 139,4 g (1,6 Mol) Morpholin werden zusammengegeben und auf Rückfluss erwärmt. Nach 22 Stunden wird abgekühlt und das Morpholin abdestilliert. Der Rückstand wird in Ether aufgenommen und mit verdünnter Salzsäure extrahiert. Die Salzsäurelösung wird alkalisch gestellt und mit Ether extrahiert. Die Etherlösung wird mit $Na_2SO_4$ getrocknet und eingeengt. Der Rückstand wird aus Ethanol umkristallisiert. Smp. 73-75°C.

$C_{14}H_{18}ClNO_2$ (267,76)

berechnet: C 62,80 % H 6,77 % N 5,23 % Cl 13,24 %

gefunden: C 63,01 % H 6,85 % N 5,33 % Cl 13,14 %

$B_4$) 1-[4-(2-Hydroxyethylthio)phenyl]-2-methyl-2-morpholino-propanon-1

20,1 g (0,075 Mol) 1-(4-Chlorphenyl)-2-methyl-2-morpholino-propanon-1 und 6,45 g (0,0825 Mol) Mercaptoethanol werden in 100 ml Dimethylformamid auf 95° C erwärmt. Dann werden 20,7 g (0,15 Mol) Kaliumcarbonat (wasserfrei) zugegeben. Die Suspension wird bei 95°C gerührt, bis keine Ausgangsverbindung mehr nachweisbar ist. Das erkaltete Reaktionsgemisch wird mit Wasser übergossen und das Produkt wird in Ether aufgenommen. Die Etherschicht wird mit Wasser gewaschen, über $Na_2SO_4$ getrocknet und eingeengt. Das erhaltene Oel wird über eine Trockensäule gereinigt und kristallisiert nach einiger Zeit.

Smp. 62-64°C.

$C_{16}H_{23}NO_3S$ (309,42)

Berechnet: C 62,11 % H 7,49 % N 4,53 % S 10,36 %

Gefunden: C 62,15 % H 7,59 % N 4,82 % S 10,49 %

In gleicher Weise werden weitere Thioetherderivate hergestellt, die der folgenden Tabelle 1 aufgeführt sind. Bei tiefsiedenden Mercaptanen wird unter Druck gearbeitet. Die erhaltenen Rohprodukte können durch Umkristallisation oder durch Chromatographie über einer Kieselgel-Mitteldrucksäule gereinigt werden (Fliessmittel: Gemische von Essigester und Hexan). Von allen Verbindungen wurde die Struktur durch ein H-NMR-Spektrum bestätigt.

**Beispiel C**

Herstellung von Mercaptophenylketonen und deren S-Alkylierung

$C_1$) 1-(4-Mercaptophenyl)-2-methyl-2-morpholino-propanon-1

451 g Natriumsulfid-Hydrat (Gehalt 32-38 % $Na_2S \approx$ 2 Mol) werden in 800 ml N-Methyl-2-pyrrolidon und 400 ml Toluol suspendiert und auf 130° erwärmt. In einem Wasserabscheider werden ca. 290 ml Wasser abgetrennt und das Toluol anschliessend abdestilliert. 100 g (0,37 Mol) 1-(4-Chlorphenyl)-2-methyl-2-morpholino-propanon-1 werden portionenweise in die warme Lösung eingetragen und die Suspension während 12 Stunden auf 140° erwärmt. Nach dem Abkühlen wird der pH des Reaktionsgemisches durch Zugabe von 6N HCl auf 6 eingestellt und die Lösung von $H_2S$ befreit. Danach wird die Lösung mit 1 Liter Wasser verdünnt und mit Diethylether extrahiert. Die Etherextrakte werden mehrmals mit 20 %iger Natronlauge ausgeschüttelt, die vereinigten wässrig-alkalischen Extrakte mit 6 N HCl neutralisiert und mit Diethylether ausgeschüttelt. Die

Etherextrakte werden über $MgSO_4$ getrocknet und eingeengt. Das verbleibende Oel wird aus Cyclohexan kristallisiert. Smp. 68 - 69° C.

$C_{14}H_{19}NO_2S$ (265,37)
Ber. C 63,37 H 7,22 N 5,28 S 12,08 %
Gef. C 63,42 H 7,31 N 5,40 S 12,05 %.

$C_2$) 1-(4-Allylthiophenyl)-2-methyl-2-morpholino-propanon-1

10 g (0,038 Mol) 1-(4-Mercaptophenyl)-2-methyl-2-morpholino-propanon-1, 5 g (0,041 Mol) frischdestilliertes Allylbromid und 5,2 g (0,038 Mol) trockenes Kaliumcarbonat werden in 50 ml Aceton suspendiert und 16 Stunden zum Rückfluss erhitzt. Nach dem Abkühlen wird das Lösungsmittel abdestilliert, der Rückstand mit Wasser und Diethylether versetzt und die Wasserphase mit Ether extrahiert. Die organische Phase wird mit 10 % NaOH-Lösung und Wasser gewaschen, über $MgSO_4$ getrocknet und eingeengt. Das zurückbleibende Oel wird im Kugelrohr destilliert bei 180 - 200° C/13 Pa. (Verbindung Nr. 23)

$C_{17}H_{23}NO_2S$ (305,47)
Ber. C 66,84 H 7,60 N 4,59 O 10,48 S 10,50 %
Gef. C 66,92 H 7,64 N 4,63 O 10,60 S 10,52 %.

**Beispiel D - S-Carbalkoxyalkylierung**

1-[4-(2-Methoxycarbonylethylthio)-phenyl]-2-methyl-2-morpholino-propanon-1

Zu einer Lösung von 10 g (0,038 Mol) 1-(4-Mercaptophenyl)-2-methyl-2-morpholino-propanon-1 und 10 Tropfen Morpholin in 50 ml trockenem Dioxan werden unter Eiskühlung 5 ml frisch destilliertes Methylacrylat zugegeben. Es wird 20 Stunden bei Raumtemperatur gerührt, mit 100 nl Diethylether verdünnt und die organische Phase mit gesättigter $NaHCO_3$-Lösung und Wasser gewaschen. Nach Trocknen über $MgSO_4$ wird das Lösungsmittel am Rotationsverdampfer abdestilliert

$C_{18}H_{25}NO_4S$ (351,50)
Ber. C 61,51 H 7,17 N 3,98 S 9,12 %
Gef. C 61,63 H 7,30 N 4,05 S 8,94 %.

**Tabelle 1**

| Verbindung Nr. | Strukturformel | Herstellungs-methode | Reinigung | Physikal. Eigenschaften |
|---|---|---|---|---|
| 1 | 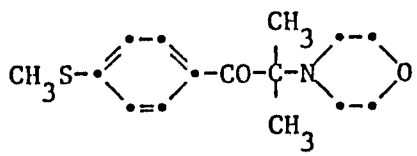 | A | Krist. (Ethanol). | Smp. 75-78° |
| 2 | 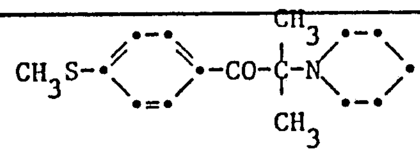 | A | Rohprodukt | Smp. 53-55° |
| 3 | 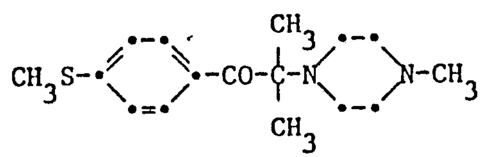 | A | Rohprodukt | Smp. 100-101° |
| 4 | 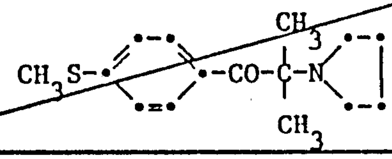 | A | Chromatographie | flüssig |
| 2 ~~5~~ | 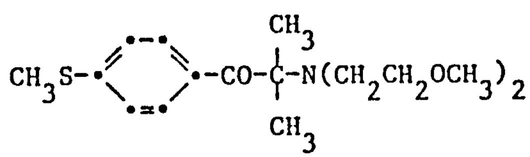 | A | Rohprodukt | flüssig |

**Fortsetzung Tabelle 1**

| Verbindung Nr. | Strukturformel | Herstellungs-methode | Reinigung | Physikal. Eigenschaften |
|---|---|---|---|---|
| 6 | 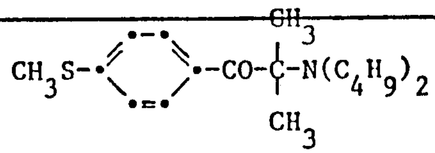 | A | Rohprodukt | flüssig |
| 7 | 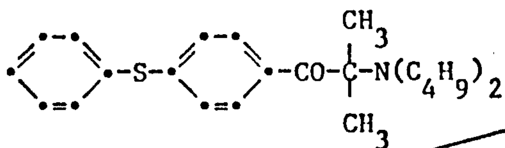 | B | Rohprodukt | flüssig |
| 8 | 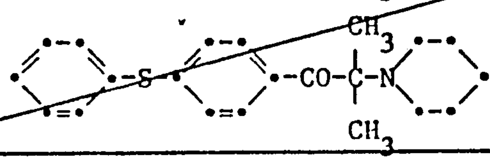 | B | Rohprodukt | Smp. 69-72° |
| 3 ~~9~~ | 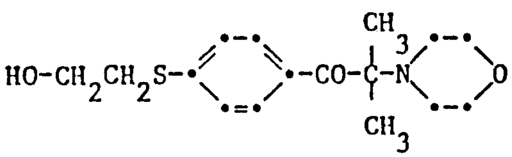 | B | Chromatographie | Smp. 62-64° |
| 4 ~~10~~ | 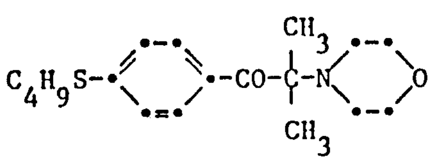 | B | Krist. (Ethanol) | Smp. 88-90° |

**Fortsetzung Tabelle 1**

| Verbindung Nr. | Strukturformel | Herstellungsmethode | Reinigung | Physikal. Eigenschaften |
|---|---|---|---|---|
| ~~11~~ | ~~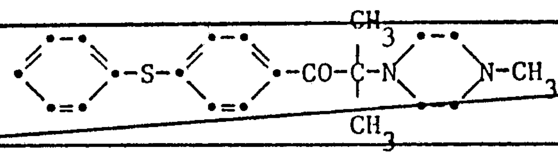~~ | ~~B~~ | ~~Krist. (Hexan)~~ | ~~Smp. 79-81°~~ |
| 5 ~~12~~ | 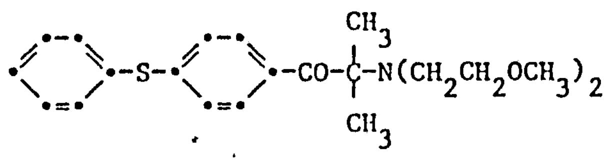 | B | Chromatographie | flüssig |
| 6 ~~13~~ | 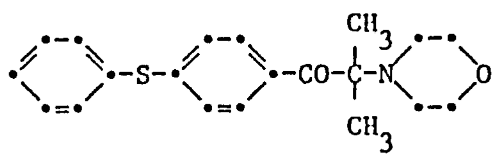 | B | Krist. (Ethanol) | Smp. 107-109° |
| 7 ~~14~~ | 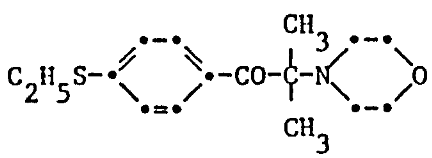 | B | Chromatographie | Smp. 67-69° |
| 8 ~~15~~ | 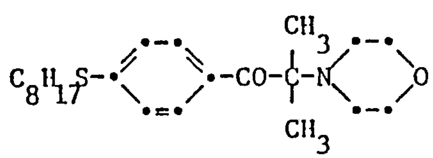 | B | Chromatographie | Smp. 69-71° |

**Fortsetzung Tabelle 1**

| Verbindung Nr. | Strukturformel | Herstellungs-methode | Reinigung | Physikal. Eigenschaften |
|---|---|---|---|---|
| 9 ~~16~~ | 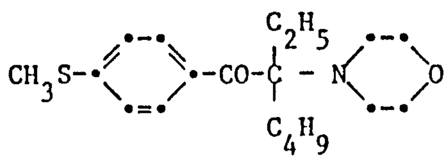 | A | Chromato-graphie | viskos |
| 17 | 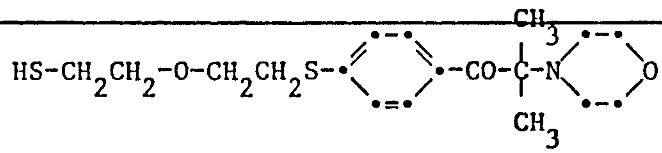 | B | Chromato-graphie | viskos |
| 18 | 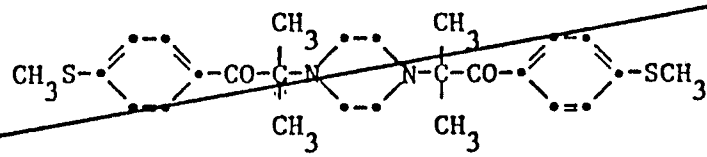 | A | Krist. (Chlf.) | Smp.188-91° |
| 10 ~~19~~ | 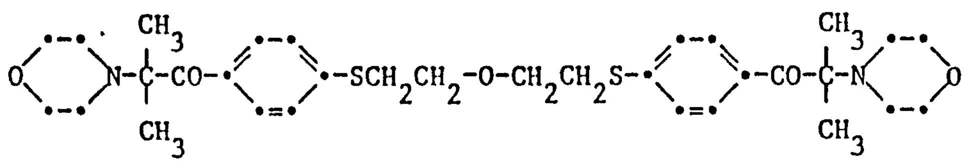 | B | Chromato-graphie | viskos |

**Fortsetzung Tabelle 1**

| Verbindung Nr. | Strukturformel | Herstellungs-methode | Reinigung | Physikal. Eigenschaften |
|---|---|---|---|---|
| 11 ~~20~~ | 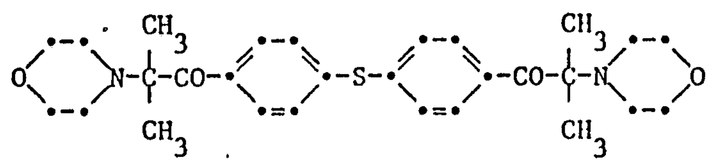 | B | Chromato-graphie | Smp. 158-160° |
| 21 | 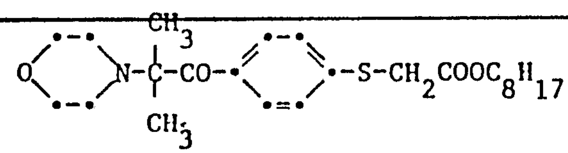 | B | Chromato-graphie | viskos |
| 22 | 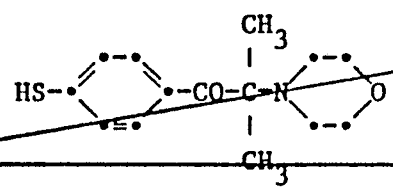 | $C_1$ | Krist. (Cyclohexan) | Smp. 68-69° |
| 12 ~~23~~ | 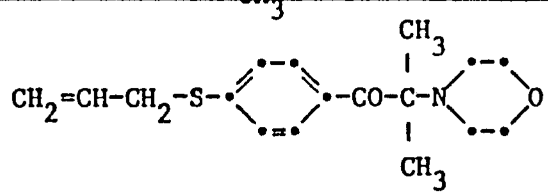 | $C_2$ | Destill. | Sdp. 180-200° 13 Pa |
| 13 ~~24~~ | 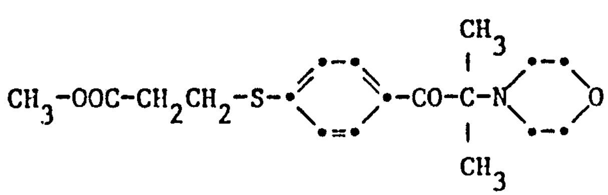 | D | Molekular-Destill. | Sdp. 180°/ 0,1 Pa |

**Fortsetzung Tabelle 1**

| Verbindung Nr. | Strukturformel | Herstellungs-methode | Reinigung | Physikal. Eigenschaften |
|---|---|---|---|---|
| 25 | 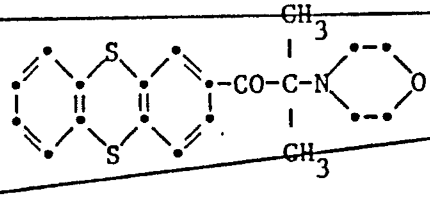 | A | Krist. (Ethylacetat) | Smp. 158-161° |
| 14 ~~26~~ | 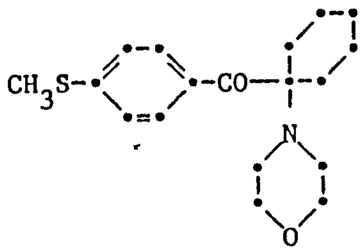 | A | Krist. (Ethanol) | Smp. 93 - 95° |
| 27 | 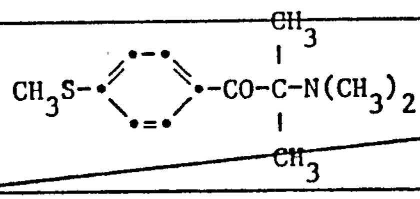 | A | Rohprodukt | flüssig |
| 15 ~~28~~ | 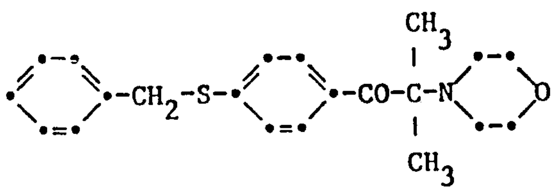 | B | Rohprodukt | Smp. 112 - 116° |

**Fortsetzung Tabelle 1**

| Verbindung Nr. | Strukturformel | Herstellungs-methode | Reinigung | Physikal. Eigenschaften |
|---|---|---|---|---|
| 16. ~~29~~ | 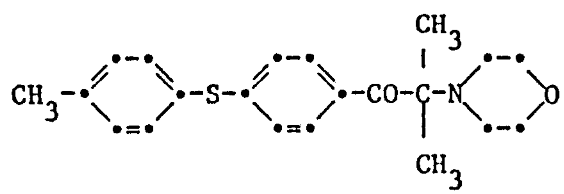 | B | Rohprodukt | Smp. 59 - 63° |
| ~~30~~ | 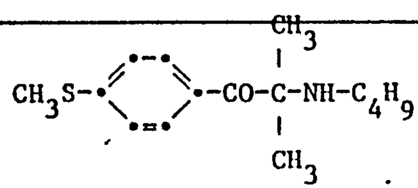 | ~~A~~ | ~~Silicagel-Filtration~~ | ~~flüssig~~ |
| ~~31~~ | 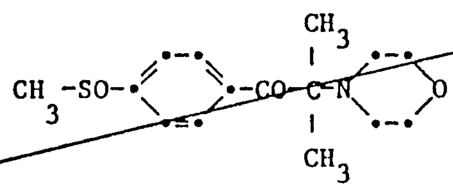 | ~~F~~ | ~~Kristall. (Hexan/Ethyl-acetat)~~ | ~~Smp. 112 -113°~~ |
| 17 ~~32~~ | 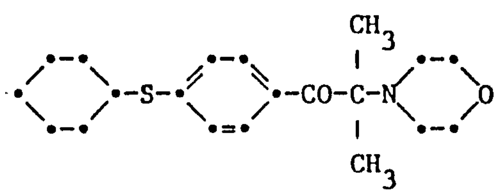 | B | Kristall. (Isopropanol) | Smp. 74 - 76° |

**Tabelle 1a — Analysen**

| Verbindung Nr. | | C | H | N | S |
|---|---|---|---|---|---|
| | ber. | 64,48 | 7,58 | 5,01 | 11,48 % |
| 1 | gef. | 64,49 | 7,51 | 5,10 | 11,51 % |
| 2 | ber. | 62,74 | 8,36 | 4,30 | 9,85 % |
| | gef. | 62,89 | 8,32 | 4,56 | 9,78 % |
| 3 | ber. | 62,11 | 7,49 | 4,53 | 10,36 % |
| | gef. | 62,15 | 7,59 | 4,82 | 10,49 % |
| 4 | ber. | 67,25 | 8,47 | 4,36 | 9,97 % |
| | gef. | 67,10 | 8,44 | 4,43 | 9,77 % |
| 5 | ber. | 68,19 | 7,54 | 3,61 | 8,27 % |
| | gef. | 67,95 | 7,47 | 3,58 | 7,87 % |
| 6 | ber. | 70,35 | 6,79 | 4,10 | 9,39 % |
| | gef. | 70,14 | 6,77 | 3,86 | 9,07 % |
| 7 | ber. | 65,50 | 7,90 | 4,78 | 10,93 % |
| | gef. | 65,65 | 7,72 | 4,83 | 10,71 % |
| 8 | ber. | 69,98 | 9,34 | 3,71 | 8,49 % |
| | gef. | 69,84 | 9,42 | 3,67 | 8,38 % |
| 9 | ber. | 68,02 | 8,71 | 4,18 | 9,56 % |
| | gef. | 68,18 | 8,71 | 4,15 | 9,59 % |
| 10 | ber. | 63,97 | 7,38 | 4,66 | 10,67 % |
| | gef. | 62,28 | 7,50 | 4,43 | 12,73 % |
| 11 | ber. | 67,71 | 7,31 | 5,64 | 6,46 % |
| | gef. | 67,75 | 7,45 | 5,75 | 6,32 % |
| 12 | ber. | 66,84 | 7,60 | 4,59 | 10,50 % |
| | gef. | 66,92 | 7,64 | 4,63 | 10,52 % |
| 13 | ber. | 61,51 | 7,17 | 3,98 | 9,12 % |
| | gef. | 61,63 | 7,30 | 4,05 | 8,94 % |
| 14 | ber. | 67,67 | 7,88 | 4,38 | 10,03 % |
| | gef. | 67,76 | 7,77 | 4,55 | 9,94 % |
| 15 | ber. | 70,95 | 7,09 | 3,94 | 9,02 % |
| | gef. | 70,68 | 7,12 | 4,05 | 8,85 % |
| 16 | ber. | 70,95 | 7,09 | 3,94 | 9,02 % |
| | gef. | 70,83 | 7,03 | 3,81 | 9,00 % |
| 17 | ber. | 69,12 | 8,41 | 4,01 | 9,22 % |
| | gef. | 69,04 | 8,64 | 4,18 | 8,99 % |

Die erfindungsgemässen pigmentierten Massen enthalten ein olefinisch ungesättigtes, photopolymerisierbares Bindemittel. Das Bindemittel kann aus einer oder mehreren ungesättigten Verbindungen bestehen, vorzugsweise enthält es zwei oder drei ungesättigte Verbindungen. Daneben kann das Bindemittel noch andere filmbildende Komponenten enthalten, die nicht ungesättigt sind und daher an der Polymerisation nicht teilnehmen. Die ungesättigten Verbindungen können eine oder mehrere olefinische Doppelbindungen enthalten. Sie können niedermolekular (monomer) oder höhermolekular (oligomer) sein. Beispiele für Monomere mit einer Doppelbindung sind Alkyl- oder Hydroxyalkyl-acrylate oder -methacrylate, wie z.B. Methyl-, Ethyl-, Butyl-, 2-Ethylhexyl- oder 2-Hydroxyethylacrylat, Isobornylacrylat, Methyl- oder Ethylmethacrylat. Weitere Beispiele hierfür sind Acrylnitril, Acrylamid, Methacrylamid, N-substituierte (Meth)acrylamide, Vinylester wie Vinylacetat, Vinylether wie Isobutylvinylether, Styrol, Alkyl- und Halogenstyrole, N-Vinylpyrrolidon, Vinylchlorid oder Vinylidenchlorid.

Beispiele für Mononere mit mehreren Doppelbindungen sind Ethylenglykol-, Propylenglykol-, Neopentylglykol-, Hexamethylenglykol-, oder Bisphenol-A-diacrylat, 4,4'-Bis(2-acryloyloxyethoxy)-diphenylpropan, Trimethylolpropan-triacrylat, Pentaerythrit-triacrylat oder -tetraacrylat, Vinylacrylat, Divinylbenzol, Divinylsuccinat, Diallylphthalat, Triallylphosphat, Triallylisocyanurat oder Tris(2-acryloyloxyethyl)isocyanurat.

Beispiele für höhermolekulare (oligomere) mehrfach ungesättigte Verbindungen sind acrylierte Epoxidharze, acrylierte Polyether, acrylierte Polyurethane oder acrylierte Polyester. Weitere Beispiele für ungesättigte Oligomere sind ungesättigte Polyesterharze, die meist aus Maleinsäure, Phthalsäure und einem oder mehreren Diolen hergestellt werden und Molekulargewichte von etwa 500 bis 3000 besitzen. Solche ungesättigte Oligomere kann man auch als Prepolymere bezeichnen.

Die Bindemittel für die erfindungsgmässen photohärtbaren Massen können z.B. ein Gemisch eines einfach ungesättigten und eines mehrfach ungesättigten Monomeren sein.

Meistens verwendet man jedoch Zweikomponenten-Gemische eines Prepolymeren mit einem mehrfach ungesättigten Monomeren oder Dreikomponentengemische, die ausserdem noch ein einfach ungesättigtes Monomer enthalten. Das Prepolymere ist hierbei in erster Linie für die Eigenschaften des Lackfilmes massgebend, durch seine Variation kann der Fachmann die Eigenschaften des gehärteten Filmes beeinflussen. Das mehrfach ungesättigte Monomere fungiert als Vernetzer, das den Lackfilm unlöslich macht. Das einfach ungesättigte Monomere fungiert als reaktiver Verdünner, mit dessen Hilfe die Viskosität herabgesetzt wird ohne dass man ein Lösungsmittel verwenden muss.

Solche Zwei- und Dreikomponentensysteme auf der Basis eines Prepolymeren werden sowohl für Druckfarben als auch für Lacke, Photoresists oder andere gefärbte photohärtbare Massen verwendet. Als Bindemittel für Druckfarben werden vielfach auch Einkomponenten-Systeme auf der Basis photohärtbarer Prepolymerer verwendet.

Ungesättigte Polyesterharze werden meist in Zweikomponentensystemen zusammen mit einem einfach ungesättigten Monomer, vorzugsweise mit Styrol, verwendet. Für Photoresists werden oft spezifische Einkomponentensysteme verwendet, wie z.B. Polymaleinimide oder Polychalkone.

Das Bindemittel kann ausserdem nicht-photopolymerisierbare filmbildende Komponenten enthalten. Diese können z.B. physikalisch trocknende Polymere bzw. deren Lösungen in organischen Lösungsmitteln sein, wie z.B. Nitrocellulose oder Celluloseacetobutyrat. Diese können aber auch chemisch bzw. thermisch härtbare Harze sein, wie z.B. Polyisocyanate, Polyepoxide oder Melaminharze. Die Mitverwendung von thermisch härtbaren Harzen ist für die Verwendung in sogenannten Hybrid-Systemen von Bedeutung, die in einer ersten Stufe photopolymerisiert werden und in einer zweiten Stufe durch thermische Nachbehandlung vernetzt werden.

Die erfindungsgemäss photohärtbaren Massen enthalten ein Pigment oder einen Farbstoff. Vorzugsweise enthalten sie ein Pigment. Das Pigment kann ein anorganisches Pigment sein, wie z.B. Titandioxid (Rutil oder Anatas), Eisenoxidgelb, Eisenoxidrot, Chromgelb, Chromgrün, Nickeltitangelb, Ultramarinblau, Kobaltblau, Cadmiumgelb, Cadmiumrot oder Zinkweiss. Das Pigment kan ein organisches Pigment sein, wie z.B. ein Mono- oder Bisazopigment oder ein Metallkomplex davon, ein Phthalocyaninpigment,ein polycyclisches Pigment, wie z.B. ein Perylen-, Thioindigo-, Flavanthron-, Chinacridon-, Tetrachlorisoindolinon oder Triphenylmethan-Pigment. Das Pigment kann auch ein Russ sein oder ein Metallpulver, wie z.B. Aluminium- oder Kupferpulver. Das Pigment kann auch ein Gemisch von zwei oder mehreren sein, wie es zur Erzielung bestimmter Farbtöne üblich ist.

Das Pigment kann in einer Menge von 5 bis 60 Gew.-%, bezogen auf die gesamte Masse, vorliegen; in Druckfarben liegen meist 10-30 % Pigment vor.

In Photoresists oder reprographischen Filmen werden zur Farbgebung statt Pigmenten auch häufig Farbstoffe verwendet. Hierbei kann es sich um organische Farbstoffe der verschiedensten Klassen handeln, beispielsweise Azofarbstoffe, Methinfarbstoffe, Anthrachinon-Farbstoffe oder Metallkomplexfarbstoffe. Diese Farbstoffe sind in den verwendet Konzentrationen in den jeweiligen Bindemitteln löslich. Die üblichen Konzentrationen sind 0,1 bis 20 %, vorzugsweise 1-5 Gew.-%, bezogen auf die gesamte Masse.

Aehnliche Probleme wie bei der Strallenhärtung von gefärbten Massen können auch bei der Strahlenhärtung von ungefärbten Massen, die einen Füllstoff enthalten, auftreten. Auch in diesen Fällen können die vorhin beschriebenen Photoinitiatoren mit Erfolg verwendet werden. Beispiele für solche Massen sind Metallprimer, Grundierungsanstriche und Spachtelmassen. Beispiele für Füllstoffe in solchen Massen sind Kaolin, Talk, Baryt, Gips, Kreide oder silikatische Füllstoffe.

Ausser den drei essentiellen Komponenten (Bindemittel, Pigment, Photoinitiator) kann die

photopolymerisierbare Masse weitere Bestandteile enthalten, die vor allem vom beabsichtigten Verwendungsgebiet abhängen. Während für die meisten Zwecke Lösungsmittelfreie Massen bevorzugt sind, kann zur Erzielung der für den Auftrag benötigten Viskosität der Zusatz eines Lösungsmittels notwendig sein. Hierzu sind die üblichen Lacklösungsmittel geeignet, die häufig Gemische verschiedener Lösungsmittel darstellen. Für einen gleichmässigen Auftrag können auch Verlaufhilfsmittel, Thixotropiemittel oder Netzmittel zugesetzt werden. Bei Druckfarben werden häufig Wachse oder sonstige Gleitmittel zugesetzt.

Obwohl die erfindungsgemässen Massen eine ausgezeichnete Dunkellagerbeständigkeit haben, kann es für bestimmte Zwecke, z.B. für die Verwendung in tropischen Ländern, sinnvoll sein, Polymerisationsinhibitoren zuzusetzen. Hierzu werden z.B. Hydrochinon und dessen Derivate, β-Naphthole, sterisch gehinderte Phenole, Kupferverbindungen, Verbindungen des dreiwertigen Phosphors, Phenothiazin, quartäre Ammoniumverbingungen oder Hydroxylaminderivate verwendet.

Umgekehrt können zur Beschleunigung der UV-Härtung oder Erreichung bestimmter physikalischer Eigenschaften Kettenübertragungsmittel wie tertiäre Amine oder Thiolverbindungen zugesetzt werden. Auch der Zusatz radikalischer Initiatoren wie von Peroxiden oder anderen organischen Perverbindungen sowie von Benzpinakol oder anderen thermisch spaltbaren organischen Verbindungen kann in bestimmten Fällen die Photopolymerisation beschleunigen.

Die erfindungsgemässen Massen können auch einen Photosensibilisator enthalten, der die spektrale Empfindlichkeit in bestimmte Bereiche verschiebt. Dies kann z.B. ein organischer Farbstoff, Perylen oder ein Derivat des Anthracens oder Thioxanthons sein. Insbesondere Thioxanthonderivate, wie z.B. Alkylthioxanthone oder Thioxanthoncarbonsäureester, bewirken als Sensibilisatoren eine starke Beschleunigung der Photopolymerisation.

Bevorzugt verwendet man als Photoinitiator nur eine Verbindung der Formel I. Für spezielle Fälle kann aber die Verwendung eines Gemisches von zwei solchen Verbindungen oder eines Gemisches mit einem anderen bekannten Photoinitiator von Vorteil sein. Die benötigte Menge Photoinitiator in der photohärtbaren gefärbten Masse beträgt 0,1 - 20 Gew.-%, vorzugsweise 1-6 Gew.-%.

Die erfindungsgemässen Massen können für verschiedene Zwecke verwendet werden. Die wichtigste und bevorzugte Verwendung ist diejenige für Druckfarben. Hierbei kann es sich un solche für Offsetdruck, Hochdruck, Tiefdruck, Siebdruck oder Flexodruck handeln. Besonders geeignet sind die erfindungsgemässen Druckfarben für den Offsetdruck, Siebdruck und Tiefdruck.

Ein zweites wichtiges Verwendungsgebiet ist die Verwendung für Anstrichstoffe. Pigmentierte Anstriche finden vor allem als Grundierung für den Korrosionsschutz von Metallen Verwendung, jedoch auch als farbige

Decklacke für dekorative Zwecke auf allen möglichen Substraten wie z.B. Metall, Holz, Pappe, Kunststoff oder Textilien. Von besonderen Interesse ist die Anwendung der erfindungsgemässen Massen für Weisslacke und für schwarzpigmentierte Metallprimer.

Weitere Verwendungsgebiete sind die Strahlenhärtung von Photoresists, die Photovernetzung silberfreier Filme oder sonstige Gebiete der Photoreproduktion.

In all diesen Verwendungen wird die photohärtbare Masse in dünner Schicht auf ein Substrat aufgetragen. Falls ein Lösungsmittel enthalten war, wird dieses anschliessend weitgehend entfernt, beispielsweise durch Erwärmen in einem Trockenofen, durch Darüberleiten von warmer Luft oder durch Infrarot- oder Mikrowellen-Bestrahlung. Die getrocknete Schicht wird dann mit kurzwelligem Licht bestrahlt, vorzugsweise mit UV-Licht vom Wellenlängenbereich 250 - 400 nm. Als Lichtquellen sind hierzu z.B. Quecksilbermitteldruck-, -hochdruckniederdruckstrahler sowie superaktinische Leuchtstoffröhren geeignet. Vorzugsweise wird die Strahlungshärtung in kontinuierlichem Verfahren durchgeführt, wobei das zu härtende Material unter der Strahlenquelle vorbeitransportiert wird. Die Transportgeschwindigkeit ist massgeblich für die Produktionsgeschwindigkeit des Artikels; sie hängt von der benötigten Bestrahlungszeit ab. Deshalb ist die Beschleunigung der Strahlenhärtung durch Photoinitiatoren ein wichtiger Faktor für die Produktion solcher Artikel und es ist einer der Vorteile der Photoinitiatoren der Formeln I, dass sie bereits in niedriger Konzentration auch bei Massen mit hohem Pigmentgehalt eine rasche Härtung gewährleisten.

Bei Verwendung eines Hybrid-Systems als Bindemittel kann die Härtung des Filmes in zwei Stufen durchgeführt werden. Beispielsweise wird durch Strahlenpolymerisation der photopolymerisierbaren Anteile ein Prepolymer geschaffen, das anschliessend durch thermische Kondensation der kondensierbaren Anteile ausgehärtet wird. Eine solche zweistufige Verfahrensweise kann z.B. für Beschichtungen oder Verklebungen von Interesse sein,sowie bei der Härtung von relativ dicken Schichten.

Ein anderes zweistufiges Verfahren ist die Kombination von Elektronenbestrahlung und UV-Bestrahlung, die ebenfalls für dickere Schichten von Interesse ist. Während die Elektronenstrahlen eine Härtung in der Tiefe des Filmes bewirken, wird die Oberfläche durch die UV-Bestrahlung gehärtet.

Die folgenden Beispiele erläutern die Eigenschaften und Anwendbarkeit der erfindungsgemässen photohärtbaren Massen. Teile sind darin Gewichtsteile, Prozente sind Gewichts-Prozente.

**Beispiel 1**:

Eine blaue Druckfarbe wird nach folgender Rezeptur hergestellt:
66 Teile Setalin® AP 560 (Urethanacrylatharz der Fa. Synthese, Holland),

11 Teile 4,4'-Di-(β-acryloyloxyethoxy)-diphenylpropan-2,2 (Ebecryl® 150, UCB, Belgien),
23 Teile Irgalithblau®GLSM (Ciba-Geigy AG, Basel)

Das Gemisch wird auf einem 3-Walzenstuhl homogenisiert und bis auf eine Korngrösse von 5 μm gemahlen.

Von dieser Druckfarbe werden jeweils 5 g mit der gewünschten Menge an Photoinitiator auf einer Tellerreibmaschine unter einem Druck von 1765,8 Pa unter Wasserkühlung homogen vermischt.

Von dieser Druckfarbe werden mit einem Probedruckgerät (Fa. Prüfbau, BR Deutschland) Offsetdrucke auf 4 x 20 cm Streifen aus Spezialpapier gemacht. Die Druckbedingungen sind:

Auflage Druckfarbe 2 g/m²
Anpressdruck 2,4525 M Pa
Druckgeschwindigkeit 2 m/s >erbei wird eine Druckwalze mit Metalloberfläche (Aluminium) verwendet.

Die bedruckten Proben werden in einem UV-Bestrahlungsgerät (QC-Processor der Fa. RPC, USA) bei einer Lampenleistung von 80 W/cm und einem Abstand zur Lampe von 11 cm bestrahlt. Die Bestrahlungszeit wird durch Variation der Transportgeschwindigkeit der Proben variiert.

Die Oberflächentrocknung der Druckfarbe wird unmittelbar nach der Bestrahlung durch den sogenannten Transfer-Test geprüft. Dabei wird ein weisses Papier unter einem Druck von 2,4525 M Pa an die bedruckte Probe angepresst. Wenn das Papier farblos bleibt, ist der Test bestanden. Wenn sichtbare Mengen Farbe auf den Teststreifen übertragen werden, so ist dies ein Zeichen, dass die Oberfläche der Probe noch nicht genügend gehärtet ist.

In der Tabelle 2 ist die maximale Transportgeschwindigkeit angegeben, bei der der Transfer-Test noch bestanden wurde.

Zur Prüfung der Durchhärtungder Druckfarbe werden ebenfalls Offset-Drucke hergestellt wie vorhin beschrieben, jedoch werden Druckwalzen mit Gummi-Oberfläche verwendet und es wird die Metallseite von Aluminium-beschichteten Papierstreifen bedruckt.

Die Bestrahlung geschieht wie oben beschrieben. Unmittelbar nach der Bestrahlung wird die Durchhärtung in einem REL-Durchhärtungsprüfgerät getestet. Dabei wird auf die bedruckte Probe ein mit Stoff überspannter Aluminium-Zylinder aufgesetzt und unter einem Druck von 21 582 Pa innerhalb 10 s einmal um die eigene Achse gedreht. Wenn dabei auf der Probe sichtbare Beschädigungen entstehen, so ist die Druckfarbe ungenügend durchgehärtet. In der Tabelle 2 wird die maximale Transportgeschwindigkeit angegeben, bei der der REL-Test noch bestanden wurde.

**Tabelle 2**

| Photoinitiator | | Maximale Transportgeschwindigkeit (m/min) | |
|---|---|---|---|
| Verbindung Nr. | Menge (Gew.-%) | Transfer-Test (Oberflächenhärtung) | REL-Test (Durchhärtung) |
| 1 | 6 | >170 | 70 |
| | 3 | 130 | 40 |
| 2 | 6 | >170 | 40 |
| | 3 | 80 | 20 |
| 3 | 6 | >170 | 50 |
| | 3 | 130 | 30 |
| 4 | 6 | 90 | 30 |
| | 3 | 60 | 20 |
| 5 | 6 | 80 | 20 |
| | 3 | 20 | 20 |
| 7 | 6 | 170 | 30 |
| | 3 | 80 | 20 |
| 8 | 6 | 60 | 30 |
| | 3 | 30 | 20 |
| 9 | 6 | >170 | 50 |
| | 3 | 130 | 40 |
| 10 | 6 | >170 | 50 |
| | 3 | 80 | 30 |
| 12 | 6 | 150 | 40 |
| | 3 | 60 | 20 |
| 14 | 6 | >170 | 40 |
| | 3 | 80 | 20 |
| 16 | 6 | >170 | 40 |
| | 3 | 70 | 20 |
| 17 | 6 | >170 | 30 |
| | 3 | 40 | 20 |

**Beispiel 2**: Mitverwendung von Thioxanthonen als Sensibilisatoren.

Es wird ein Weisslack nach folgender Rezeptur hergestellt:
17,6 g Ebecryl® 593 (Polyesteracrylatharz der Fa. UCB, Belgien),
11,8 g N-Vinylpyrrolidon
19,6 g Titandioxid RTC-2 (Titandioxid der Fa. Tioxide, England),
19,6 g Sachtolit®HDA (Lithopone der Sachtleben Chemie, BRD)
11,8 g Trimethylolpropan-trisacrylat,
19,6 g Setalux®UV 2276 (acryliertes Epoxidharz auf der Basis von Bisphenol A, Kunstharzfabrik Synthese, Holland).

Die obigen Komponenten werden zusammen mit 125 g Glasperlen (Durchmesser 4 cm) in einer 250 ml Glasflasche während mindestens 24 Stunden auf eine Korngrösse ≤ 5 μm gemahlen.

Die so erhaltene Stammpaste wird in Portionen geteilt und jede Portion mit den in der Tabelle 3 angegebenen Photoinitiatoren und Photosensibilisatoren (Co-Initiatoren) durch Einrühren bei 60° C gemischt und die Mischungen nochmals 16 Stunden mit Glasperlen gemahlen.

Die so hergestellten Weisslacke werden in einer Dicke von 30 μm mit einem Rakel auf Glasplatten aufgetragen. Die Proben werden in einem PPG-Bestrahlungsgerät mit einer Lampe von 80 W/cm in einem Durchgang belichtet. Dabei wird die Geschwindigkeit des Durchganges der Proben durch das Bestrahlungsgerät laufend gesteigert bis keine ausreichende Härtung mehr eintritt. Die maximale Geschwindigkeit, bei der noch ein wischfester Lackfilm entsteht, ist in Tabell 3 als "Härtungsgeschwindigkret" angegeben. >erbei werden folgende Verbindungen verwendet:

PI 1 = Verbindung Nr. 1 der Tabelle 1
PS 1 = 2-Isopropylthioxanthon
PS 2 = 2-Dodecylthioxanthon
PS 3 = 2-Methyl-6-ethoxycarbonyl-thioxanthon
PS 4 = Verbindung der Formel

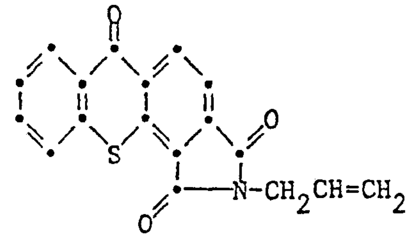

PS 5 = Verbindung der Formel

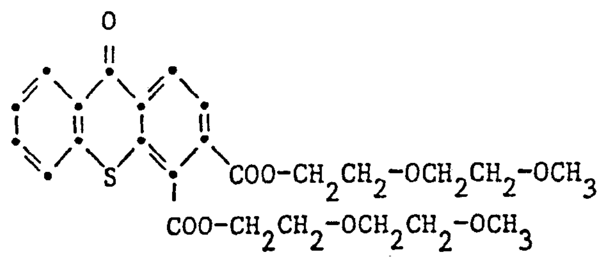

PS 6 = 2-Methoxy-6-ethoxycarbonyl-thioxanthon.

**Tabelle 3**

| Photoinitiator | Co-Initiator (Sensibilisator) | Härtungsgeschwindigkeit |
|---|---|---|
| 2 % PI 1 | — | 10 m/min. |
| — | 0,5 % PS 3 | 10 m/min. |
| 2 % PI 1 | 0,25 % PS 3 | 70 m/min. |
| 2 % PI 1 | 0,25 % PS 4 | 90 m/min. |
| 2 % PI 1 | 0,25 % PS 5 | 60 m/min. |
| 2 % PI 1 | 0,25 % PS 6 | 70 m/min. |

Man ersieht daraus, dass bereits kieine Mengen des Sensibilisators die Härtungsgeschwindigkeit wesentlich beschleunigen.

## Patentansprüche

1. Photohärtbare gefärbte Masse, enthaltend
a) ein olefinisch ungesättigtes, photopolymerisierbares Bindemittel,
b) ein Pigment oder einen Farbstoff und
c) einen Photoinitiator dadurch gekennzeichnet, dass der Photoinitiator eine Verbindung der Formel 1 ist,

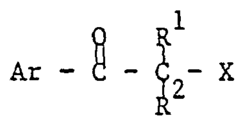

I

worin Ar ein durch die Gruppe -S-$R^9$ substituierter Phenylrest ist, $R^9$ Wasserstoff, $C_1$-$C_8$-Alkyl, $C_3$-$C_6$-Alkenyl, Cyclohexyl, Benzyl, Phenyl, Tolyl oder eine der Gruppen $-CH_2CH_2OH$, $-CH_2CH_2-OOC-CH=CH_2$, $-CH_2-COO(C_1-C_4\text{-Alkyl})$, - $CH_2CH_2-COO(C_1-C_4\text{-Alkyl})$,

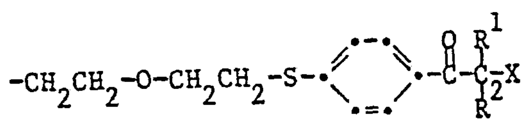

oder

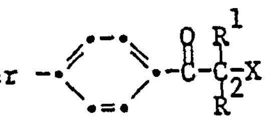

bedeutet,
$R^1$ und $R^2$ $C_1$-$C_4$-Alkyl oder $R^1$ und $R^2$ zusammen $C_4$-$C_5$-Alkylen bedeuten und
X ein Morpholinorest oder ein Rest der Formel $-N(CH_2CH_2-OCH_3)_2$ist.

2. Zusammensetzung gemäss Anspruch 1, dadurch gekennzeichnet, dass der Photoinitiator eine Verbindung der Formel 1 ist, worin Ar 4-Mercapto-phenyl, 4-Methylthiophenyl oder 4-(2-Hydroxyethyl)thiophenyl ist, $R^1$ und $R^2$ unabhängig voneinander Methyl, Ethyl oder Butyl sind und X eine Morpholinogruppe ist.

3. Zusammensetzung gemäss Anspruch 1, enthaltend zusätzlich als Sensibilisator ein Thioxanthon-Derivat.

4. Verwendung einer Masse gemäss Anspruch 1 bis 3 als Druckfarbe.

5. Verfahren zur photochemischen Härtung von gefärbten Massen, die ein olefinisch ungesättigtes, photopolymerisierbares Bindemittel, ein Pigment und einen Photoinitiator enthalten durch Bestrahlung mit kurzwelligem Licht, dadurch gekennzeichnet, dass man als Photoinitiator eine Verbindung der Formel I gemäss Anspruch 1 verwendet.

## Claims:

1. A photocurable coloured composition containing
a) an olefinically unsaturated, photopolymerisable binder,
b) a pigment or a dye and
c) a photoinitiator,
in which composition the photoinitiator is a compound of formula I

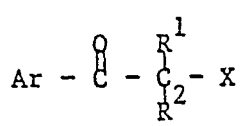

I

wherein Ar is a phenyl radical which is substituted by the group -S-$R^9$, $R^9$ is hydrogen, $C_1$-$C_8$alkyl, $C_3$-$C_6$alkenyl, cyclohexyl, benzyl, phenyl, tolyl or one of the groups $-CH_2CH_2OH$, $-CH_2CH_2-OOC-CH=CH_2$, $-CH_2-COO(C_1-C_4alkyl)$, $-CH_2CH_2-COO(C_1-C_4alkyl)$,

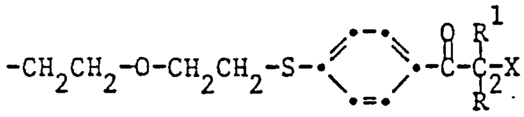 or 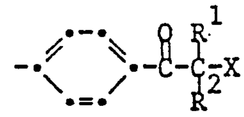

$R^1$ and $R^2$ are $C_1$-$C_4$alkyl or $R^1$ and $R^2$ together are $C_4$-$C_5$-alkylene and X is a morpholino radical or a radical of the formula $-N(CH_2CH_2-OCH_3)_2$.

2. A composition according to claim 1, wherein the photoinitiator is a compound of formula I, wherein A is 4-mercaptophenyl, 4-methylthiophenyl or 4-(2-hydroxyethyl)-thiophenyl, each of $R^1$ and $R^2$ independently of the other is methyl, ethyl or butyl and X is a morpholino group.

3. A composition according to claim 1, which additionally contains as sensitiser a thioxanthone derivative.

4. Use of a composition according to any one of claims 1 to 3 as printing ink.

5. A process for the photochemical curing of coloured compositions containing an olefinically unsaturated photopolymerisable binder, a pigment and a photoinitiator by irradiation with shortwave light, which process comprises the use of a compound of formula 1 according to claim 1 as the photoinitiator.

**Revendications**

1. Composition colorée photodurcissable, contenant:
a) un liant photopolymérisable, à insaturation oléfinique,
b) un pigment ou un colorant, et
c) un photo-amorceur,
caractérisée en ce que le photo-amorceur est un composé de formule I:

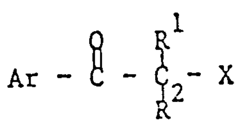 I

dans laquelle Ar représente un reste phényle substitué par le groupe $-S-R^9$,
$R^9$ représente un atome d'hydrogène, un groupe alkyle en $C_1$ à $C_8$, alcényle en $C_3$ à $C_6$, cyclohexyle, benzyle, phényle, tolyle ou l'un des groupes $-CH_2CH_2OH$, $-CH_2CH_2-OOC-CH=CH_2$, $-CH_2-COO$(alkyle en $C_1$ à $C_4$), ($CH_2CH_2-COO$(alkyle en $C_1$ à $C_4$),

Fl( 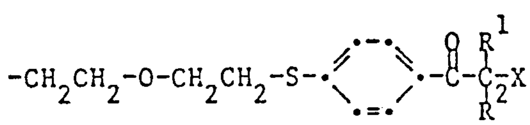 ou 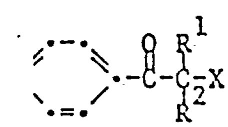

$R^1$ et $R^2$ représentent chacun un groupe alkyle en $C_1$ à $C_4$ ou bien $R^1$ et $R^2$ forment ensemble un groupe alkylène en $C_4$ ou $C_5$, et
X représente un reste morpholino ou un reste de formule $-N(CH_2CH_2-OCH_3)_2$.

2. Composition selon la revendication 1, caractérisée en ce que le photo-amorceur est un composé de formule I, dans laquelle Ar représente un groupe mercapto-4 phényle, mé-thylthio-4 phényle ou (hydroxy-2 éthyl)-4 thiophényle, $R^1$ et $R^2$ représentent chacun, indépendamment l'un de l'autre, un groupe méthyle, éthyle ou butyle, et X est un groupe morpholino.

3. Composition selon la revendication 1, contenant en outre comme sensibilisant un dérivé de thioxanthone.

4. Utilisation d'une composition selon l'une des revendications 1 à 3 comme encre d'impression.

5. Procédé pour le durcissement photochimique de compositions colorées, qui contiennent un liant photopolymérisable à insaturation oléfinique, un pigment et un photoamorceur, par irradiation par de la lumière à courtes longueurs d'ondes, procédé caractérisé en ce qu'on utilise comme photoamorceur un composé de formule I selon la revendication 1.